Europäisches Patentamt

European Patent Office

Office européen des brevets

(19)

(11) Numéro de publication: **0 327 461**

**A1**

(12) **DEMANDE DE BREVET EUROPEEN**

(21) Numéro de dépôt: 89400296.3

(22) Date de dépôt: 02.02.89

(51) Int. Cl.⁴: **A 61 K 37/14**

(30) Priorité: 02.02.88 FR 8801176

(43) Date de publication de la demande:
09.08.89 Bulletin 89/32

(84) Etats contractants désignés:
AT BE CH DE FR GB IT LI LU NL SE

(71) Demandeur: ROUSSEL-UCLAF
35, boulevard des Invalides
F-75007 Paris (FR)

(72) Inventeur: Brossard, Claudine
5, rue Paul Doumer
F-95520 Osny (FR)

Quero, Anne-Marie
9, rue de Malabry
F-92350 Le Plessis Robinson (FR)

Smets, Pierre
137, rue du Maréchal Leclerc
F-78670 Villennes sur Seine (FR)

(74) Mandataire: Vieillefosse, Jean-Claude et al
Roussel-Uclaf 111, route de Noisy B.P. 9
F-93230 Romainville (FR)

(54) Utilisation des protéines du lait pour la fabrication d'un médicament antiviral.

(57) Utilisation des protéines du lait, notamment celles capables de fixer le fer, pour la fabrication d'un médicament antivital, utile dans le traitement des affections à virus enveloppés ainsi que des affections à virus non enveloppés.

EP 0 327 461 A1

**EP 0 327 461 A1**

## Description

### Utilisation des protéines du lait pour la fabrication d'un médicament antiviral

La présente invention a pour objet l'utilisation des protéines du lait, notamment celles capables de fixer le fer, pour la fabrication d'un médicament antiviral.

Les protéines du lait, notamment celles capables de fixer le fer, d'origines animale ou humaine sont connues depuis de nombreuses années pour leurs propriétés antibactériennes. De nombreux procédés d'obtention de ces protéines ont été décrits dans la litterature. On peut notamment citer l'article de CHERON paru dans (C.R. Acad. Sc Paris t.284 14 Février 1977) et les demandes de brevets français N¤ 2505615 et 2584727.

En poursuivant l'étude des protéines du lait, notamment celles capables de fixer le fer, la demanderesse a découvert de manière tout à fait inattendue que celles-ci présentaient en outre de remarquables propriétés antivirales ; propriétés qui, à la connaissance de la demanderesse n'avaient jamais été mises en évidence jusqu'alors.

La présente demande a ainsi pour objet l'utilisation des protéines du lait, notamment celles capables de fixer le fer, pour la fabrication d'un médicament antiviral.

L'invention a également pour objet l'utilisation des protéines du lait, notamment celles capables de fixer le fer, pour la fabrication d'un médicament inhibant les virus enveloppés.

L'invention a plus particulièrement pour objet l'utilisation des lactotransferrines pour la fabrication d'un médicament inhibant les virus enveloppés.

L'invention a également pour objet l'utilisation des protéines du lait, notamment celles capables de fixer le fer, pour la fabrication d'un médicament inhibant les virus non enveloppés.

L'invention a plus particulièrement pour object l'utilisation des lactotransferrines pour la fabrication d'un médicament inhibant les virus non enveloppés.

En raison de leurs remarquables propriétés antivirales illustrées plus loin dans la partie expérimentale, les médicaments antiviraux renfermant les protéines du lait, notamment celles capables de fixer le fer, selon l'invention, peuvent être administrés à titre préventif ou curatif. Ils trouvent leur emploi dans le traitement des affections virales, notamment dans le traitement des affections à virus enveloppés, ainsi que dans le traitement des affections à virus non enveloppés.

Les médicaments selon l'invention peuvent ainsi être utilisés à titre de médicaments inhibant notamment les HERPESVIRIDAE [virus de l'herpes simplex type 1 et 2 (HSV1 et HSV2, cytomégalovirus] ou les RHABDOVIRIDAE (virus de la stomatite vésiculeuse VSV).

Les médicaments selon l'invention peuvent aussi être utilisés à titre de médicaments inhibant notamment les RHINOVIRUS.

Ces médicaments peuvent ainsi être utilisés dans le traitement des affections à Rhinovirus affectant les voies aériennes supérieures de l'homme.

La dose usuelle, variable selon le produit utilisé, le sujet traité et l'affection en cause, peut varier de 0,1 mg à 50 mg par jour chez l'homme, en fonction de la voie utilisée.

Dans le cas des lactotransferrines on peut par exemple administrer chaque jour des compositions pharmaceutiques renfermant de 1 à 10 mg de principe actif par voie cutanée chez l'homme ; un tel traitement devant alos être répété pendant plusieurs jours, voire plusieurs semaines.

A titre de médicaments, les produits définis ci-dessus, peuvent être incorporés dans des compositions pharmaceutiques destinées à la voie digestive, parentérale ou locale.

Ces compositions pharmaceutiques peuvent être, par exemple, solides ou liquides et se présenter sous les formes pharmaceutiques couramment utilisées en médecine humaine, comme par exemple, les comprimés, simples ou dragéifiés, les gélules, les granulés, les sirops, les aérosols, les collutoires, les collyres, les suppositoires, les ovules, les préparations injectables, les pommades, les crèmes, les lotions ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les protéines du lait, notamment celles capables de fixer le fer, peuvent être préparées par exemple comme indiqué dans la demande de brevet français publiée sous le N¤ 2.584.727.

Il va être donné maintenant à titre non limitatif, des exemples de mise en oeuvre de l'invention.

**Exemple 1 :**

On a préparé des aérosols délivrant des doses contenant chacune :

| | |
|---|---|
| - Lactotransferrine | 2 mg |
| - Emulsifiant | 0,15 mg |
| - Propulseur | 50 mg |

2

**Exemple 2 :**
On a préparé un collutoire renfermant :

- Lactotransferrine         50 mg
- Excipient aqueux qsp    100 mg

**Exemple 3 :**
On a préparé des comprimés renfermant :

- Lactotransferrine         5 mg
- Excipient (lactose,       100 mg
talc, amidon, stéarate
de magnésium qs pour
un comprimé terminé à

## ETUDE DE L'ACTIVITE ANTIVIRALE DE LA LACTOTRANSFERRINE

I - L'activité de la lactotransferrine a été étudiée vis à vis de virus appartenant aux HERPESVIRIDAE, (HSV1, HSV2, CMV murin) et aux RHABDOVIRIDAE (VSV).
Deux méthodes ont été utilisées :
- Réduction de l'effet cytophathique produit par les virus
ou
- Réduction du nombre et de la taille des plages formées par les virus.

**REDUCTION DE L'EFFET CYTOPATHIQUE**

### 1. MATERIELS ET METHODES

Virus
Virus de l'herpes type 1 et type 2 (HSV1 et HSV2) cultivés sur cellules VERO. Virus de la stomatite vésiculeuse (VSV) cultivé sur cellules L 929.

Méthode

Les essais sont réalisés en microplaques de 96 cupules sur des cellules en tapis établi de 24 heures. La lactotransferrine en solution à 6 mg/ml est diluée de 3 en 3 (2 mg/ml - 0,666 mg/ml 0,222 mg/ml).
Les cellules sont infectées par le virus (50 DICC 50) par un contact de 10 mn. Puis 0,1 ml des dilutions de la lactotransferrine est ajouté.
Des témoins "cellules", "virus", "cytotoxicité du produit" sont effectués.
Les microplaques sont placées pendant 72 h à 37 ¤ C sous une atmosphère enrichie de 5 % de $CO_2$. L'effet cytopathique est évalué au microscope inversé selon une échelle de 0 à 4 :
0 tapis cellulaire intact
4 tapis cellulaire complètement détruit.

### 2. RESULTATS
Les concentrations de lactotransferrine réduisant de 50 % l'effet cytopathique des virus sont de :
0,222 mg/ml pour l'herpes type 1 et type 2.
0,666 mg/ml pour le virus de la stomatite vésiculeuse.

**REDUCTION DU NOMBRE ET DE LA TAILLE DES PLAGES**

### 1. MATERIELS ET METHODES

- Herpes virus type 1
Les cellules VERO sont cultivées pendant 24 heures dans une plaque à 24 puits. Le tapis cellulaire, est infecté pendant 1 heure par une suspension virale d'herpes virus type 1 donnant 10 à 30 plages comptables par cupule. La suspension virale est alors éliminée et le tapis cellulaire infecté est recouvert par un milieu contenant 1,6 % de tragacanth et la lactotransferrine à la concentration finale voulue (2 mg/ml - 0,666 mg/ml 0,222 mg/ml). Les cupules "témoins virus" reçoivent du milieu sans lactotransferrine.

Les cupules "témoins cellules" ne reçoivent que du milieu de culture.

Après 5 jours à 37 ¤ C sous atmosphère enrichie de 5 % de $CO_2$, le milieu est éliminé et le tapis cellulaire est lavé puis les cellules sont fixées avec une solution de formol à 10 % et colorées par une solution de bleu de méthylène et rincées.

Les plages peuvent être observées et comptées à l'oeil nu ou à la loupe binoculaire ou au microscope inversé.

- Cytomégalovirus murin

Le protocole opératoire est légèrement différent du précédent. La suspension virale est placée sur la couche cellulaire constituée de fibroblastes de souris pendant 1 h à 37 ¤ C puis éliminée, la solution de lactotransferrine à la concentration étudiée est alors placée sur les cellules infectées pendant 1 heure à 37 ¤ C puis éliminée. La couche cellulaire est alors recouverte d'un milieu contenant 1,6 % de tragacanth. Après 5 jours d'incubation à 37 ¤ C en atmosphère enrichie de 5 % de $CO_2$, la lecture est effectuée à la loupe binoculaire ou au microscope inversé.

## 2. RESULTATS

Pour l'herpes virus type 1 la concentration de lactotransferrine qui réduit de 50 % le nombre des plages est de 2 mg/ml. Une concentration de 0,22 mg/ml réduit encore la taille des plages.

Pour le cytomégalovirus murin la concentration de lactotransferrine qui réduit de 50 % le nombre des plages est de 0,66 mg/ml.

## CONCLUSION

La lactotransferrine présente une activité antivirale "in vitro" sur des virus appartenant aux Herpesviridae (HSV1, HSV2, CMV murin) et aux Rhabdoviridae (VSV).

II - L'activité de la lactotransferrine a également été étudiée vis à vis de virus appartenant à la famille des RHINOVIRUS.

La méthode utilisée consiste à déterminer l'activité inhibitrice de la lactotransferrine vis à vis de différentes souches de Rhinovirus en déterminant la réduction du nombre et de la taille des plages.

## 1. MATERIELS ET METHODES.

Matériel
- Lactotransferrine : solution mère à 12,5 mg/ml.
- Lignée diploide de fibroblastes humains embryonnaires MRC5, cultivée en milieu M3 : MBE, HEPES, 1% SVF, Peni-Sm.
- Souches virales :
Rhinovirus prototypes : RV5, RV9, RV31.
Rhinovirus sauvages : RV88/a, RV88/b, RV88/c, RV88/d, RV88/e.

Méthode
- Culture en microplaques de 96 puits de 24 à 48 heures.
- Eliminer le milieu de culture.
- Inoculer 100 μl de suspension virale par puits : surnageant d'une culture présentant 90% d'ECP dilué au 1/10 en milieu M3.
- Incuber 60 minutes à 37 ¤.
- Aspirer l'inoculum et ajouter par puits 150 μl de M3 contenant le produit testé aux concentrations de 0,125 mg/ml, 0,25 mg/ml, 0,5 mg/ml, 1,25 mg/ml.

## 2. RESULTATS.

Les résultats obtenus avec les différentes souches de Rhinovirus figurent dans le tableau ci-après :

| Réf. souche | Concentrations en mg/ml | | | | Témoin virus |
|---|---|---|---|---|---|
| | 0,125 | 0,25 | 0,5 | 1,25 | |
| RV5 | + + | + | 0 | nf | + + |
| RV9 | + | 0 | 0 | nf | + + |
| RV31 | + | +/- | 0 | nf | + |
| RV88/a | 0 | 0 | 0 | nf | + + |
| RV88/b | + + | + | + | +/- | + + |
| RV88/c | + + | + + | + | 0 | + + |
| RV88/d | + + | + + | + + | nf | + + |
| RV88/e | + | 0 | 0 | nf | + |

4

**CONCLUSION**

Ces résultats montrent clairement que la lactotransferrine exerce une activité inhibitrice sur le Rhinovirus à une concentration voisine de 0,25 mg/ml.

**Revendications**

1) Utilisation des protéines du lait, notamment celles capables de fixer le fer, pour la fabrication d'un médicament antiviral.

2) Utilisation des protéines du lait, notamment celles capables de fixer le fer, pour la fabrication d'un médicament pouvant être employé dans le traitement des affections à virus enveloppés.

3) Utilisation des protéines du lait, notamment celles capables de fixer le fer, pour la fabrication d'un médicament inhibant les virus enveloppés.

4) Utilisation des lactotransferrines pour la fabrication d'un médicament inhibant les virus enveloppés.

5) Utilisation des dérivés tels que définis à l'une quelconque des revendications 1 à 4, pour le fabrication d'un médicament inhibant les HERPESVIRIDAE.

6) Utilisation des dérivés tels que définis à l'une quelconque des revendications 1 à 4, pour la fabrication d'un médicament inhibant les RHABDOVIRIDAE.

7) Utilisation des protéines du lait, notamment celles capables de fixer le fer, pour la fabrication d'un médicament pouvant être exploité dans le traitement des affections à virus non enveloppés.

8) Utilisation des protéines du lait, notamment celles capables de fixer le fer, pour la fabrication d'un médicament inhibant les virus non enveloppés.

9) Utilisation des lactotransferrines pour la fabrication d'un médicament inhibant les virus non enveloppés.

10) Utilisation des dérivés tels que définis à l'une quelconque des revendications 1 et 7 à 9, pour la fabrication d'un médicament inhibant les Rhinovirus.

11) Utilisation des dérivés tels que définis à l'une quelconque des revendications 1 à 4, pour la fabrication d'un médicament inhibant les virus de l'herpes simplex type 1 et 2, (HSV1 et HSV2), cytomégalovirus.

12) Utilisation des dérivés tels que définis à l'une quelconque des revendications 1 à 4, pour la fabrication d'un médicament inhibant le virus de la stomatite vésiculeuse, VSV.

13) Utilisation des dérivés tels que définis à l'une quelconque des revendications 1 et 7 à 9, pour la fabrication d'un médicament permettant de traiter les affections à Rhinovirus affectant les voies aériennes supérieures de l'homme.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | BIOLOGICAL ABSTRACTS, vol. 85, no. 6, 1988, résumé ref. no. 55402, Philadelphia, PA, US; G. HANGOC et al.: "Modulation of Friend virus infectivity in vivo by administration of purified preparations of human lactoferrin and recombinant murine interleukin-3 to mice", & LEUKEMIA (BALTIMORE) 1(11): 762-764. 1987 * Résumé * | 1 | A 61 K  37/14 |
| X | BIOLOGICAL ABSTRACTS, vol. 84, no. 8, 1987, résumé ref. no. 78996, Philadelphia, PA, US; L. LU et al.: "Protective influence of lactoferrin on mice infected with the polyeythomia-inducing strain of Friend virus complex", & CANCER RES. 47(5):4184-4188. 1987 * Résumé * | 1 | |
| X | BIOLOGICAL ABSTRACTS, vol. 83, no. 11, 1987, résumé ref. no. 109686, Philadelphia, PA, US; L.T. CHEN et al.: "Effects of purified iron-saturated human lactoferrin on spleen morphology in mice infected with Friend virus complex", & AM J. PATHOL 126(2): 285-292. 1987 * Résumé * | 1 | DOMAINES TECHNIQUES RECHERCHES (Int. Cl.4)  A 61 K |
| Y,D | FR-A-2 505 615  (SOCIETE NATIONALE ELF AQUITAINE) * Page 1, lignes 6-27 * | 1 | |
| Y,D | FR-A-2 584 727  (ROUSSEL-UCLAF) * Page 3, lignes 17-23 * -/- | 1 | |

Le présent rapport a été établi pour toutes les revendications

| Lieu de la recherche | Date d'achèvement de la recherche | Examinateur |
|---|---|---|
| LA HAYE | 26-04-1989 | TURMO Y BLANCO C.E. |

Office européen
des brevets

**RAPPORT DE RECHERCHE EUROPEENNE**

Numero de la demande

EP  89 40 0296

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y,D | C.R. ACAD. SC. PARIS, vol. 284, série D, 14 février 1977, pages 585-588; A. CHERON et al.: "BIOCHIMIE. - Fractionnement chromatographique et études sur la microhétérogénéité de la lactotransferrine de Vache préparée par un procédé orginal"<br>* Résumé *<br>----- | 1 | |
| | | | **DOMAINES TECHNIQUES RECHERCHES (Int. Cl.4)** |

Le présent rapport a été établi pour toutes les revendications

| Lieu de la recherche | Date d'achèvement de la recherche | Examinateur |
|---|---|---|
| LA HAYE | 26-04-1989 | TURMO Y BLANCO C.E. |

CATEGORIE DES DOCUMENTS CITES

X : particulièrement pertinent à lui seul
Y : particulièrement pertinent en combinaison avec un
autre document de la même catégorie
A : arrière-plan technologique
O : divulgation non-écrite
P : document intercalaire

T : théorie ou principe à la base de l'invention
E : document de brevet antérieur, mais publié à la
date de dépôt ou après cette date
D : cité dans la demande
L : cité pour d'autres raisons
............................................................
& : membre de la même famille, document correspondant

EPO FORM 1503 03.82 (P0402)